# EUROPEAN PATENT SPECIFICATION

(11) **EP 4 164 481 B1**
(45) Date of publication and mention of the grant of the patent: **16.10.2024**
(21) Application number: 21734529.7
(22) Date of filing: 10.06.2021
(51) Int. Cl.: A61B 5/08, G01N 33/00, G01N 33/497, A61B 5/097

(54) **A DEVICE FOR MEASURING VOLATILE MARKERS IN BREATH**
VORRICHTUNG ZUR MESSUNG FLÜCHTIGER MARKER IN ATEM
DISPOSITIF DE MESURE DE MARQUEURS VOLATILS DANS L'AIR EXPIRÉ

(30) Priority: 11.06.2020 US 202063037822 P; 15.03.2021 US 202163161117 P
(43) Date of publication of application: 19.04.2023
(73) Proprietor: Nanose Medical Ltd, 3009000 Isfiya (IL)
(72) Inventor: HAICK, Hossam, 3537511 Haifa (IL); MAROM ALBECK, Orit, 4676746 Herzliya (IL); SELLA TAVOR, Osnat, 1933000 Kfar Kisch (IL)
(74) Representative: Fleuchaus & Gallo Partnerschaft mbB
(86) International application number: PCT/IL2021/050701
(87) International publication number: WO 2021/250674

(56) References cited:
- WO-A1-2017/216794
- US-A1- 2015 293 057
- PANTALEI SIMONE ET AL: "Improving sensing features of a nanocomposite PEDOT:PSS sensor for NO breath monitoring", SENSORS AND ACTUATORS B: CHEMICAL, ELSEVIER BV, NL, vol. 179, 23 October 2012 (2012-10-23), pages 87 - 94, XP029001316, ISSN: 0925-4005, DOI: 10.1016/J.SNB.2012.10.015
- SANTOS JOSÉ P. ET AL: "Hand Held Electronic Nose for VOC Detection", CHEMICAL ENGINEERING TRANSACTIONS, 30, 181-186, 20 September 2012 (2012-09-20), pages 1 - 6, XP055836885, Retrieved from the Internet <URL:https://www.cetjournal.it/index.php/cet/article/view/CET1230031> [retrieved on 20210901], DOI: 10.3303/CET1230031
- WEBSTER JASON ET AL: "TruffleBot: Low-Cost Multi-Parametric Machine Olfaction", 2018 IEEE BIOMEDICAL CIRCUITS AND SYSTEMS CONFERENCE (BIOCAS), 19 October 2018 (2018-10-19), pages 1 - 4, XP055836891, ISBN: 978-1-5386-3603-9, Retrieved from the Internet <URL:http://rosenstein.engin.brown.edu/Webster_TruffleBot_BioCAS2018_preprint.pdf> DOI: 10.1109/BIOCAS.2018.8584767

## Description

### TECHNOLOGICAL FIELD

The invention generally concerns a device for determining presence and/or concentration of volatile materials in breath samples.

### BACKGROUND

Electronic noses are typically engineered and operated to sniff vapors from a sample and provide a set of measurements that are compared to stored patterns for known chemical species for identification of the sniffed vapor. The inability of gas sensors to respond differently to different chemical species makes such noses less attractive. Inclusion of a pattern recognizer allows selective analysis of responses received from every one of the different sensors in a sensor array. The pattern recognizer evaluates the responses and through predetermined, programmed, or learned patterns ascertains the chemical species effect on the sensor.

The electronic nose can match complex samples with subjective endpoints such as odors or flavors and thus can also be used as a production tool to maintain quality over long periods of time.

One of the more important aspects of electronic noses is their inability to provide an immediate, accurate and reliable reading when the samples to be tested are breath samples. Devices intended for such prognostic medicinal uses are expensive, complex and tend to provide unreliable results.

**Various sensors for analyzing gaseous samples are known in the prior art but are limited for the reasons mentioned above. A PEDOT:PSS based sensor for** nitric oxide NO breath monitoring as a bio-marker for oxidative stress including asthma **is described by** S. Pantalei et al. (Sens. Actuators, B (2013), 179, 87-94**).** In this article an exhaled air sample can be stored in a fixed volume storage chamber and recirculated over the PEDOT:PSS sensor, via a recirculation path made of inert and non-adsorbent material, until the sensor exposed to the sample reaches a dynamic equilibrium with it. **Also,** document US 2015/293057 A1 discloses a sensor for detecting target compounds such as volatile organic compounds wherein the sensor can include a nanotube array including a plurality of metal oxide nanotubes functionalized with at least one metal ion which is capable of binding with the target compounds; in some embodiments, the sample gas can be recirculated across the nanotubes, such that the target compounds will have additional opportunity to bind with the metal ions. And document WO 2017/216794 A1 discloses a system for diagnosing, screening or monitoring a disease in a test subject, the system comprising: (a) a selected definitive sensor set comprising at least three sensors reactive to the presence of volatile organic compounds (VOCs) in an exhaled breath of the test subject, and (b) a processing unit comprising a pattern recognition analyzer.

### GENERAL DESCRIPTION

The inventors of the technology disclosed herein have developed a sniff-on device as of claim 1 capable of providing accurate and on the spot analysis of gaseous samples. The device of the invention is specifically engineered for collecting and measuring content of any gaseous sample, such as a breath or an air sample from a human or animal subject, or from an environment and determine with high accuracy at least one parameter relating to the sample content. The at least one parameter enables determination of a subject's health condition, presence of a volatile organic compound (VOC), presence of a gaseous or airborne toxic material and so forth. As will be demonstrated further hereinbelow, such a sampling and measurement may be completed within seconds to minutes from real-time measurement onset.

As the collection of subject's (human or animal) breath requires, in most instances, participation from the side of the subject, the device provides the ability to withdraw a sufficient volume of a breath sample through the subject's oral cavity with or without needing subject's participation. Thus, a device according to the invention may be utilized not only with subjects who can cooperate, but also with subjects who are incapable of cooperation (such as illed subjects, disabled, the elderly or young subjects).

As a person of art would appreciate, a device according to the invention may further be utilized in determining presence of a volatile material in a surrounding or, whether contained or opened to the environment, which may attest to the condition or state of the surrounding. Such surroundings may be facilities for handling food, fruits, vegetable and so forth, as well as sewage and industrial facilities which may emit toxic volatile materials.

The invention provides a device for determining content of at least one volatile compound (VC) in a gaseous sample, e.g., a non-alveolar or an alveolar breath sample from a subject, the device comprising an array of sensors and a pattern-recognizer. The device is further configured for being adapted to one or more collection or sampling units, chambers or devices, which are configured to receive and hold a volume of the gaseous sample to be evaluated. Thus, a device of the invention generally comprises two distinct regions: a sample collection region and a sensing region. The device is configured to permit collecting sample from a sample source and flowing of the sample from the sample collection region onto the sensing region or directly from the inlet of the device onto the sensing region. In most general terms, a device of the invention comprises at least two detachable parts wherein a first part comprises a sample collection chamber and a second part comprises at least one sensor assembly. The sample collection chamber is provided with an inlet and an outlet. The sensor assembly section comprises an inlet and an outlet. The two sections are associated with each other via a channel assembly that is configured to direct a sample contained in said sample collection chamber to the at least one sensor assembly. The sample thereafter may exit the sensing assembly section through an outlet or may be flown back into the sample collection chamber and circulated back once and again Generally speaking, a device may be operated in different modes:
- an in-direct mode- wherein a sample is collected in a sample collecting chamber and flown from the chamber onto the sensor assembly;
- a direct mode- wherein a sample is flown directly onto the sensor assembly, bypassing the sample collection chamber;
- a single mode- wherein sample flown in a direct or in-direct mode, as above, is flown over the sensor assembly without recirculation; and
- a continuous mode- wherein sample flown in an in-direct mode, as above, is circulated over the sensor assembly more than one time, over a predefined period of time or in a predetermined number of cycles. The device of the claimed invention can be operated in a continuous mode.

Generally speaking, where the operation mode of the device involves the sample collecting chamber, the sample may be collected into the chamber while the chamber is disconnected from the sensing region. In such cases, the sample may be collected in one place; e.g., in an industrial environment, and its content measured after assembling the device in another place. The collection chamber being of a material and shaped in a way to permit storage of the sample for several seconds to several hours, allows using thereof while connected to the sensing region or disconnected therefrom.

The device is configured as a handheld device, and may also be configured as a partially disposable device and/or as a device for immediate or on-the-spot real-time analysis of VC sample content.

The device comprises:
- an inlet element for receiving the sample, e.g., a breath sample;
- at least one sample collecting chamber, configured to allow capturing the sample, permitting device operation in a continuous mode;
- at least one sensor assembly; and
- at least one channel assembly and pump operable to direct said sample from an inlet for receiving the sample to the at least one sample collecting chamber and the at least one sensor assembly.

According to the invention, a sample obtained from a subject or collected by other means from an environment, as disclosed herein, is received or communicated through an inlet into the at least one sample collecting chamber or directly onto the sensor assembly through a communication channel or an assembly of such channels or conduits. When the sample is collected, the sample collecting chamber permits direct circulation of the sample over the sensor assembly. Where the collecting chamber is not connected to the device, the collecting chamber may be associated with the device only after a gaseous sample has been collected thereinto.

The sample collection chamber can be made of a flexible material or rigid material. It may be of any shape and size.

Thus, in a device of the invention, the gaseous sample is allowed to continuously circulate back to and from the collection chamber and over the sensor assembly in order to maintain a stable and continuous measurement. This closed loop circulation mode permits increased exposure of the sensor assembly to the VC or analyte to be detected.

In order to cleanse or clear the collecting chamber from impurities (solids or gases) that may be present, the first sample volume collected may be exhausted out from the chamber via an outlet tube, allowing a further volume of the sample to be collected in the collecting chamber and subsequently analyzed. Alternatively, instead of cleansing the collecting chamber with a sample of the same source as the sample to be measured, air may be flown into the chamber. The air may be atmospheric air or an inert gas or a gas of high purity.

The chamber may be further used to separate between different aliquots of the sample. This may be achieved, for example, by utilizing a series of collecting chambers, wherein each separate sample may be directed to a separate chamber. A chamber containing a sample aliquot to be analyzed may be flown over the sensor assembly. All undesired samples may be evacuated via an outlet that may optionally include a filtering means or a filter assembly or a membrane.

Flow of a sample may be controlled by a set of valves (one or more) that are configured and operable, manually, mechanically or electronically, to allow or prevent air flow into and out from the collecting chamber. It is further described, a pair of valves is provided for each of said collecting chambers, wherein one valve is provided at the entrance to the chamber and another at the outlet end of the chamber. Another valve may be used to control flow of the sample from the chamber and into the sensing region or vice versa. Each valve may have an ON/OFF state, wherein in the ON state, the valve is open and allows flow of the sample, and an OFF state wherein the valve does not permit flow of the sample.

The valve positioned at the inlet end is operable, e.g., to prevent contamination of the chamber internal surface by ambient air which may comprise accidental VCs before analysis begins. However, in some cases, the valve may be operable to permit flow of atmospheric air into the device (into and through the collecting chambers and optionally also over the sensors) in order to obtain a background signal. The valve may be opened to accept a volume of the breath sample automatically when the device is set to ON, when a first volume is flown through the inlet end or when operated. Thus, when the device is inoperable, the valve may be set to position OFF.

The description also states, the device is not provided with valves, but may be provided with other means for controlling flow of gases into and from a chamber.

Where multiple (two or more) chambers are present, each of the at least one collecting chambers may be the same or different and may be positioned at substantially the same part of the device. In some embodiments, some of the at least one collecting chambers are positioned separately from some of the others. To normalize a breath sample to be analyzed, some initial data relating to the room air sample or to the breath sample are collected and analyzed in advance of the breath sample analysis. The room air/initial breath volume obtained from the subject is directed to one or more of the collecting chambers that act as "***environment testing chamber(s)***"*.* These environment testing chambers are adapted with one or more sensors that provide an initial reading of various environmental parameters relating to the breath/room sample. These include, for example, gases composition, mainly carbon dioxide, humidity, sample temperature and others.

Once the environmental parameters are collected, the chamber may be evacuated and a valve positioned at the opening of the environment testing chamber(s) is operated to prevent a further breath sample to enter the environment testing chamber. Alternatively, immediately following collection of the environmental parameters, the breath samples is flown uninterruptedly into the chamber. The further sample may be communicated/directed to the same chamber or to another of the collecting chambers and thereafter to a sensor chamber where one or more (an array, or an assembly) sensors are provided. The breath sample may be flown over the one or more sensors during the assaying of the sample or may be contained in the sensing chamber until analysis is completed. It is further described, a breath sample may be caused to flow over the sensors by the action of a pump or, generally speaking, by holding the sensor chamber under negative pressure (vacuum).

As with any of the chambers of the device, the sensing region may similarly be set with a pair of valves which can be opened or closed during operation of the device. The sensing region may independently from the collection chamber be provided with an inlet for directing a sample thereto, thereby by passing the sample chamber. The sensing region may also be provided with an outlet for removing any sample entering the sensing region, as will be further detailed hereinbelow.

Any of the devices of the invention may further comprise a pattern recognition algorithm to determine presence and/or amount of said VC in the sample.

Data communication with the sensors is achievable via a data processing unit that is in data communication with a data user interface unit; wherein the data processing unit comprising data relating to a control data set and is adapted to receiving from the sensor(s) information relating to presence of VCs or pattern thereof and provide an indication of presence or absence of one or more VCs, toxic material, disease state and other parameters as may be required.

A device according to the invention is provided as a two-part or a three-part or a multi-part device comprising detachable parts, wherein the sensor chamber is detachable, and the sample collection chamber is separately detachable. Depending on the actual device configuration, any of the detachable parts may be reusable or disposable. Factors determining which of the parts is reusable or disposable depend, *inter alia*, on the complexity of the device part, the ability to regenerate or reuse the device part, its price of manufacture, and others.

The device may further optionally comprise one or more securing mechanisms, that hold the two parts of the device together during operation or when in storage. The securing mechanism may be mechanical, e.g., in the form of a magnet or a magnetized surface, or a mechanical assembly that provide the whole device in secure form.

As described in the application, the device may further comprise means for connecting the disposable part to a multiuse part. Further, the connection is via a connector element or assembly positioned between the disposable part and the multi-use part.

In a two- or three- or multi-part device according to a comparative example, one of the parts may comprise the means for collecting the sample and a respective inlet; while another part may comprise the at least one sensor assembly and the at least one sample collecting chamber. The at least one communication means operable to direct a sample from the inlet to the at least one sample collecting chamber, and/or the at least one sensor assembly may be parted such that one portion thereof is present in the first part of the device and the other in the second part of the device. The two parts of the communication means may be fitted to provide a secure and continuous gaseous communication upon attachment of the two device parts.

However, according to the invention, there is provided a hand-held device for determining content (presence and/or amount) of at least one volatile compound (VC) in a sample, e.g., a breath sample, the device comprising at least two detachable parts, wherein a first part comprises a sample collecting chamber; and a second part comprises at least one sensor assembly. The device further comprises at least one channel assembly configured to direct said sample from the collecting chamber to the at least one sensor assembly, and to circulate said sample over the at least one sensor assembly; and
an analyzer configured for real-time analysis of the VC content in the sample;
wherein optionally one or both of said parts being disposable.

The channel assembly is associated directly or indirectly with a pump enabling circulating the sample over the sensor assembly.

Alternatively, in a comparative example, the sensor assembly could be provided separate from the other features and elements of the device.

In certain implementations of the device of the invention, the detachable part of the device containing the sensor assembly may be disposable. In other implementation the part of the device containing the sensor assembly may be detachable to enable surface effective regeneration of the sensor assembly so that the device may be reused.

It is further described, the device comprises an electrical sensor comprising at least one electrode assembly and a reader. In other implementations, the electrode assembly may be replaced with an antenna.

The sensor of the invention may be any sensor for measuring/detecting components of exhaled breath for achieving a determination of a VC profile from breath samples as described herein. Generally speaking, sensors can include functionalized surface regions (wherein such surfaces are functionalized with metal nanoparticles, functional molecules, hollow fibers and others), sensors having a functionalized nanowire or a nanotube, a polymer-coated surface acoustic wave (SAW) sensors, sensor employing a semiconductor gas sensor technology, aptamer biosensors, amplifying fluorescent polymer (AFP) sensors and others.

In accordance with the present invention, the sensor may be commercially referred to as an "artificial nose" or as an "electronic nose" which can non-invasively measure at least one VC in the exhaled breath and/or monitor the concentration of at least one VC in the exhaled breath of a subject as described herein. Thus, the herein described sensors enable qualitative and/or quantitative analysis of volatile compounds (e.g. gases, vapors, or odors) hence facilitates the device to carry out a method of the invention.

It is further described, the device comprises one or more (or an array) of chemically sensitive sensors and a processing unit comprising a learning and pattern recognition analyzer configured for receiving sensor output signals and comparing the signals to a stored data, by utilizing a pattern recognition algorithm.

It is further described, the device may be a device disclosed in International Publication No. WO 2009/144725.

It is further described, the device utilizes a sensor as disclosed in US 2011/0269632.

According to the claimed invention, the sensor is provided in the form of a plurality of nanoparticles that are associated to a surface. The sensor surface comprises a plurality of sensing regions, each of the regions being associated with same or different population of nanoparticles, such that a signal may be independently derived from each of the sensing areas, and be indicative of an interaction (or lack thereof) between VCs present in the sample and the nanoparticles on the sensing regions.

In some embodiments, each of the sensing regions present on the sensor surface comprises a plurality of nanoparticles of a particular population, wherein each population differs from another in at least one of particle size, particle morphology (e.g., core/shell particles, non-core/shell particles, spherical, cubic, tetrahedral, triangular, dumbbell, elongated, multiparticles or fused particles, etc), particle composition (e.g., doping, metallic particles, non-metallic particles, conductive particles, novel metal particles, hybrid materials, etc), surface decoration (e.g., presence of material islands, association with ligand groups, etc) and others.

It is further described, each sensing region comprises a different selection of nanoparticles. In some embodiments, each sensing region comprises a mixed population (an inhomogeneous population) of nanoparticles, while in other embodiments, each sensing region comprises a uniform population (a homogenous population) of nanoparticles.

In a plurality of such sensing regions, one or more thereof may comprise a plurality of particle populations, namely an inhomogeneous population of particles, wherein some of the nanoparticles differ in structure, others in composition and still others in surface decoration. For example, a sensing region may comprise two populations of nanoparticles, one population comprising particles of one metal and another population comprises particles of a different metal. In a similar way, all particles may be of one metal but differ from each other in their surface decoration (e.g., presence of ligands or selection of ligands).

According to the inventors, the nanoparticles are core/shell particles, non-core/shell particles, spherical, cubic, tetrahedral, triangular, dumbbell, elongated or fused particles. In some embodiments, the particles are spherical in shape.

It is further described, the nanoparticles are metallic nanoparticles; wherein the metal is optionally selected amongst any metal of the Periodic Table of the Elements. The metals are of any of Groups IIIB, IVB, VB, VIB, VIIB, VIIIB, IB and IIB of block d of the Periodic Table. The inventors inform, the metal is selected from Sc, Ti, V, Cr, Mn, Fe, Ni, Cu, Y, Zr, Nb, Tc, Ru, Mo, Rh, W, Au, Pt, Pd, Ag, Au, Al, Mn, Co, Cd, Hf, Ta, Re, Os, Ir and Hg.

It is further described, the metal is gold, silver, nickel, cobalt, copper, palladium, platinum or aluminum. Further, the nanoparticles are gold nanoparticles.

The metallic nanoparticles may or may not be doped or further comprise an amount of another metallic or non-metallic material. The metallic nanoparticles may be bare, namely uncoated, or coated with a plurality of surface associated ligand molecules. Such ligand molecules may have surface anchoring groups which may vary based on, e.g., the composition of the nanoparticles. For example, where the nanoparticles are gold nanoparticles, the surface anchoring groups may be a thiol, a disulfide, an amine and others as known in the art.

It is further described a method of using a device according to the invention, the method comprising obtaining a breath sample from a subject by employing any non-invasive means known in the art and directing or permitting flow of said breath sample into the device of the invention.

Within the context of the present invention, ***volatile compounds*** (VC) are any compounds that may be present in a gaseous sample to be tested or evaluated and which detection or monitoring is desired. In the context of medical applications, the VCs are compounds that are associated with the metabolism, presence and/or growth of at least one pathogen (e.g. bacteria or virus) or involved in the pathogenesis of another disease or disorder. The compounds may be further associated with the presence and/or evolution of a disease state not associated with a pathogen. Such disease states may be cancers, inflammatory conditions and others. VCs that are generated in the body, e.g., through the metabolism of cells or pathogens within the body, are excreted through the exhaled breath. Some of the VCs are first released into the circulatory system and thereafter excreted through the exhaled breath. The VCs may comprise a plurality of compounds, some of which gaseous, others may be liquids (at a physiological temperature), which are released into the exhaled breath and carrier by the breath gases or small droplets of water, and thus can be detected and quantified. In some cases, the VCs are volatile organic compounds (VOCs), namely such compounds that are volatile or otherwise carried in the breath and are regarded as organic compounds.

The "***VC profile***" or VOC profile refers to the breath signature of the disease, namely to a collection of properties relating to the VC content of the exhaled breath obtained from a subject. These collective properties are unique and informative, thus may be regarded as a fingerprint or a signature indicating onset, evolution or progression of a certain disease over another. The VC profile differentiating one disease over the other can also provide an insight as to the state of the disease or the progression thereof, can identify the onset of the disease at an early stage before symptoms develop and can assist in determining success of a therapeutic treatment (prophylaxis or treatment of existing symptoms). The properties may be one or more of:
- presence or absence of one or more VCs indicative of the disease,
- the concentration (or amount) of the one or more VCs,
- the presence or absence of other VCs in combination,
- the ratio amounts between the various VCs, and
- a change in the presence or amount of one or more VCs over time.

As used herein, a "***control***" which the VC profile is compared to is any component of a VC profile obtained from subjects not having the disease to be detected, namely subjects who have been tested and found not to have been affected by the disease, or known to be free of the disease, as well as from subjects who are suffering from the disease or other diseases not detected in the specific detection session. These may be used to define a "healthy group" namely a group of subjects who do not have the specific disease that should be detected, and a "sick group", namely a group of subjects who are suffering from the specific disease to be detected. When comparing the VC profile to a VC profile of a control, a determination can be made whether the VC profile is indicative of a subject that has contracted the disease or a subject who has not.

In a similar fashion, to enable a diagnostic determination as to whether a treatment modality has been effective in reducing side effects of the disease, has ameliorated such effects, has improved the subject's health condition or has treated the disease, the VC profile taken from the subject may be compared to a control sample(s) obtained from the same subject at one or different time points prior to or after treatment has commenced.

Control samples obtained for the purpose of determining the presence or absence of a disease are typically taken from a plurality of subjects which have been identified as healthy or as sick. The number of subjects may be at least 10, 20, 30, 40, 50, 60, 70, 80, 90, 100, 150, 200, 250 to thousands of subjects.

Where determining progression of the disease is aimed, one or more VC profiles may be obtained for a group of subjects suffering from a disease, wherein each profile is obtained at a different time point along the way to recovery.

Where the subject is a human, the control is a human, and where the method if used on non-human mammals, the control group should include species from the same group.

As noted therein, a change in the VC profile, e.g., as compared to a control, may be determined by utilizing an algorithm such as, but not limited to, artificial neural networks, multi-layer perception (MLP), generalized regression neural network (GRNN), fuzzy inference systems (FIS), self-organizing map (SOM), radial bias function (RBF), genetic algorithms (GAS), neuro-fuzzy systems (NFS), adaptive resonance theory (ART) and statistical methods including, but not limited to, principal component analysis (PCA), partial least squares (PLS), multiple linear regression (MLR), principal component regression (PCR), discriminant function analysis (DFA) including linear discriminant analysis (LDA) or cluster analysis including nearest neighbor.

Using such algorithms or others known in the art, a VC profile may be regarded as significantly different and thus be indicative of any one of presence or absence of one or more VCs indicative of the disease, concentration (or amount) of the one or more VCs, presence or absence of other VCs in combination, ratio amounts between the various VOCs and a change in the presence or amount of one or more VCs over time. The term **"*significantly different*"** as used herein generally refers to a quantitative difference in the concentration or level of each VC from the set or combinations of VCs as compared to the levels of VCs in control samples obtained from, e.g., individuals not having the disease. A statistically significant difference can be determined by any test known to the person skilled in the art. Common tests for statistical significance include, among others, t-test, ANOVA1 Kruskal-Wallis, Wilcoxon, Mann-Whitney and odds ratio. Individual samples (of unknown status) can be compared with data from the reference group (negative control). An increase or decrease in the level as compared to a control or reference value or mean control level or reference value, or a change, difference or deviation from a control or reference value, can be considered to exist if the level differs from the control level or reference value, by about 5 percent or more, by about 10 percent or more, by about 20 percent or more, or by about 50 percent or more compared to the control level or reference value. Statistical significance may alternatively be calculated as P<0.05. Methods of determining statistical significance are known and are readily used by a person of skill in the art. In a further alternative, increased levels, decreased levels, deviation, and changes can be determined by recourse to assay reference limits or reference intervals. These can be calculated from intuitive assessment or non-parametric methods. Overall, these methods calculate the 0.025, and 0.975 fractiles as 0.025*(n+1) and 0.975*(n+1). Such methods are well known in the art. The presence of a VOC marker which is absent in a control, is also contemplated as an increased level, deviation or change. The absence of a VC marker which is present in a control, for example, is also contemplated as a decreased level, deviation or change.

Various other algorithms are known in the art, which are disclosed, for example, in US Patent Nos. 6,411,905, 6,606,566, 6,609,068, 6,620,109, 6,767,732, 6,820,012 and 6,839,636.

The following lists aspects of the disclosure, the invention being defined by the appended claims.

Also provided is a device for determining a content of at least one volatile compound (VC) in a breath sample obtained from a subject, the device comprising
- inlet for collecting the breath sample;
- at least one breath sample collecting chamber;
- at least one sensor assembly;
- at least one channel assembly operable to direct said breath sample from the inlet to the at least one sample collecting chamber, and/or the at least one sensor assembly; and

an analyzer configured for real-time analysis of the VC content in the sample;
wherein the at least one sensor assembly is disposable or regenerable.

A device is also provided for determining a content of at least one volatile compound (VC) in a breath sample obtained from a subject, the device comprising
- at least one breath sample collecting chamber for holding a breath sample obtained from a subject;
- at least one sensor assembly;
- at least one channel assembly operable to expose the at least one sensor assembly to the sample; and

an analyzer configured for real-time analysis of the VC content in the sample;
wherein the at least one sensor assembly is disposable or regenerable.

Another device is provided for determining a content of at least one volatile compound (VC) in a breath sample obtained from a subject, the device comprising
- inlet for collecting the breath sample from the subject's oral cavity;
- at least one breath sample collecting chamber configured and operable for holding an aliquot of the breath sample;
- at least one sensor assembly configured and operable for continuous operation;
- at least one channel assembly operable to direct said breath sample from the inlet to the at least one sample collecting chamber, and/or the at least one sensor assembly; and

an analyzer configured for real-time analysis of the VC content in the sample;
wherein the at least one sensor assembly is disposable or regenerable.

A further device is provided for determining a content of at least one volatile compound (VC) in a breath sample obtained from a subject, the device comprising
- inlet for collecting the breath sample;
- at least one breath sample collecting chamber;
- at least one sensor assembly;
- at least one channel assembly operable to direct said breath sample from the inlet to the at least one sample collecting chamber, and/or the at least one sensor assembly; and

an analyzer configured for real-time analysis of the VC content in the sample;
wherein the at least one sensor assembly is disposable or regenerable.

Also provided is a hand-held device for determining presence of a volatile compound (VC) in or content of at least one gaseous sample, the device comprising at least two optionally detachable parts, wherein a first part comprises a sample collecting chamber; and a second part comprises at least one sensor assembly; wherein
the sample collecting chamber and the at least one sensor assembly are in gaseous communication;
the device further comprising a closed loop channel assembly and a pump configured to direct said sample from the sample collecting chamber to the at least one sensor assembly and to circulate said sample from the sample collecting chamber over the at least one sensor assembly over a period of time; and
an analyzer configured for real-time analysis of the VC presence in or content of the sample;
wherein the at least one sensor assembly comprises one or a plurality of sensing regions and wherein optionally one or more of said at least two optionally detachable parts are disposable.

In some embodiments, the sample is a breath sample. In some embodiments, the breath sample is obtained from a subject and is received through an inlet provided in the sample collecting chamber.

It is further described, the at least one collecting chamber is configured to separate between different aliquots of the sample.

Further, the device comprises a valve or a valve assembly configured and operable, manually, mechanically or electronically, to allow or prevent air follow into the collecting chambers or out of the chambers.

In some embodiments, the device comprises two or more sample collecting chamber, wherein one or more of the sample collecting chambers is an environment testing chamber adapted with one or more sensors providing an initial reading of environmental parameters.

In some embodiments, the one or more sensor are configured for providing a reading relating to any one of gas composition, carbon dioxide presence and concentration, humidity and sample temperature.

In some embodiments, the device comprises a data processing unit for data communication with the sensor assembly; a data user interface unit being in data communication with the data processing unit; wherein the data processing unit comprising data relating to a control data set and is adapted to receiving from the sensor(s) information relating to presence of VCs or pattern thereof and provide an indication of presence or absence of one or more VCs, and disease state.

In some embodiments, one or more of said at least two optionally detachable parts is disposable.

It is further described, the at least one sensor assembly comprises a sensor in the form of a functionalized surface region, a sensor having a functionalized nanowire or a nanotube, a polymer-coated surface acoustic wave (SAW) sensors, sensor employing a semiconductor gas sensor technology, aptamer biosensors, or amplifying fluorescent polymer (AFP) sensor.

It is further described, the at least one sensor assembly comprises one or more chemically sensitive sensors and a processing unit comprising a learning and pattern recognition analyzer configured for receiving sensor output signals and comparing the signals to a stored data, by utilizing a pattern recognition algorithm.

In some embodiments, the sensor is provided in the form of a plurality of nanoparticles associated to a surface.

In some embodiments, the sensor surface comprises one or more sensing regions, each of the regions being associated with same or different population of nanoparticles, wherein a signal independently derived from each of the sensing areas is indicative of an interaction (or lack thereof) between VCs present in the sample and the nanoparticles on the sensing regions.

It is further described, each of the sensing regions comprises a plurality of nanoparticles of a particular population, wherein each population differs from another in at least one of particle size, particle morphology, particle composition, and surface decoration.

Further, each of the sensing regions comprises a different selection of nanoparticles.

Further, each of the sensing regions comprises a mixed population.

In some embodiments, VCs are associated with the metabolism, presence and/or growth of at least one pathogen or involved in the pathogenesis of a disease or disorder.

In some embodiments, the device is for determining a VC profile selective and indicative of onset, evolution or progression of a disease state.

In some embodiments, the VC profile differentiating one disease over another, provides an indication of a disease state or progression thereof, identifies onset of a disease at a stage before symptoms develop and/or determine success of a therapeutic treatment.

It is further described, device comprises at least two detachable parts.

In some embodiments, the sample collecting chamber is configured to receive a gaseous sample while disconnected from the at least one sensor assembly.

It is further described, the sample collecting chamber is provided under vacuum.

Further, the sample collecting chamber is configured to connect to a pump.

In some embodiments, the closed loop channel assembly having at least one outlet operable to exhaust the sample upon demand.

A hand-held device is further provided for determining presence of a volatile compound (VC) in or content of at least one gaseous sample, the device comprising at least two optionally detachable parts, wherein a first part comprises a sample collecting chamber; and a second part comprises at least one sensor assembly; wherein
the sample collecting chamber and the at least one sensor assembly are in gaseous communication;
the device further comprising a channel assembly and a pump configured to direct said sample from the sample collecting chamber to the at least one sensor assembly; and
an analyzer configured for real-time analysis of the VC presence in or content of the sample;
wherein the at least one sensor assembly comprises one or a plurality of sensing regions and wherein optionally one or more of said at least two detachable parts are disposable.

Also provided is a hand-held device for determining presence of a volatile compound (VC) in or content of at least one gaseous sample, the device comprising optionally a sample collecting chamber in gaseous communication with at least one sensor assembly;
the at least one sensor assembly being provided with an inlet for receiving the at least one gaseous sample and an outlet for releasing said sample; and a channel assembly and a pump configured to direct said sample from the inlet to flow over the at least one sensor assembly; and
an analyzer configured for real-time analysis of the VC presence in or content of the sample;
wherein the at least one sensor assembly comprises one or a plurality of sensing regions.

Also described is a method for detecting at least one VC in a gaseous sample, the method comprising exposing a sensor assembly comprising at least one sensing region with a sample comprising at least one VC or suspected of containing same and using a pattern recognition algorithm to determine presence and/or amount of said VC in the sample.

The inventors inform, the sensor assembly is as defined herein.

The inventors found, exposing the sensor is by flowing the gaseous sample over the sensor assembly one or more times.

It is further described, exposing the sensor is by allowing said gaseous sample to flow from a collecting chamber containing said sample one or more times, continuously, or over a period of time.

It is further described, exposing the sensor is by directing a gaseous sample directly onto the sensor assembly.

The device comprises a sample collecting chamber, at least one sensor assembly, at least one channel assembly configured to direct a gaseous sample from the collecting chamber to the at least one sensor assembly and to circulate said sample over the at least one sensor assembly and an analyzer configured for real-time analysis of the VC content in the sample.

In such a device, the gaseous sample may be flown directly from the device inlet or mouthpiece onto the sensor assembly, without being communicated through or stored in a collecting chamber. In such away, the sample is flown over the sensor assembly and thereafter exhausted out of the device (through an outlet). To permit a continuous and repeated exposure of the sensor assembly to the sample, a sample may be stored in a collecting chamber and recirculated once and again over the sensor assembly.

In some aspects, the method is for detecting at least one volatile compound (VC) in a gaseous sample, the method comprising exposing a sensor assembly comprising at least one sensing region to the sample comprising at least one VC or suspected of containing same and using a pattern recognition algorithm to determine presence and/or amount of said VC in the sample.

In some embodiments, the sensor assembly is provided in a device according to the invention.

It is further described, exposing the sensor assembly comprises flowing the gaseous sample over the sensor assembly one or more times.

Further, exposing the sensor assembly comprises allowing said sample to flow from a collecting chamber containing said sample one or more times, continuously, or over a period of time.

Further, exposing the sensor assembly comprises directing the sample directly onto the sensor assembly.

Further provided is a method for detecting at least one volatile compound (VC) in a gaseous sample, the method comprising providing a hand-held device comprising a sample collecting chamber and at least one sensor assembly to capture the sample in said sample collecting chamber, and exposing said sensor assembly comprising at least one sensing region to the sample comprising at least one VC or suspected of containing same; wherein
the sample collecting chamber and the at least one sensor assembly are in gaseous communication;
the device further comprising a closed loop or an open-loop channel assembly and a pump configured to direct said sample from the sample collecting chamber to the at least one sensor assembly and to optionally circulate said sample from the sample collecting chamber over the at least one sensor assembly over a period of time; and
using a pattern recognition algorithm to determine presence and/or amount of said VC in the sample.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** provides an illustration of a device according to the invention.
**Figs. 2A-B** provide an illustration of the open (**Fig. 2A**) and closed-loop (**Fig. 2B**) circulation modes of devices according to the invention.
**Fig. 3** provides graphical results of measures made with devices according to the invention.
**Figs. 4A-C** provide three embodiments of devices according to the invention.
**Figs. 5A-C** provide an illustration of sensor arrays (sensor assembly) with different exemplary arrangements of sensing regions.

### DETAILED DESCRIPTION

As disclosed herein, the invention is defined by the appended claims and concerns a device for detecting at least one volatile material, in a gaseous sample. The gaseous sample may be any gas-state sample which comprises a gaseous material or an air-borne material or another material that is soluble in the gaseous sample or humidity present therein. Notwithstanding the origin and composition of the sample, a device of the invention are capable of providing an indication as to the presence of a material in the sample (or suspected of being contained therein) and/or the amount thereof. A device of the invention also enable on-the-spot determination of a state of the origin from which the sample was obtained.

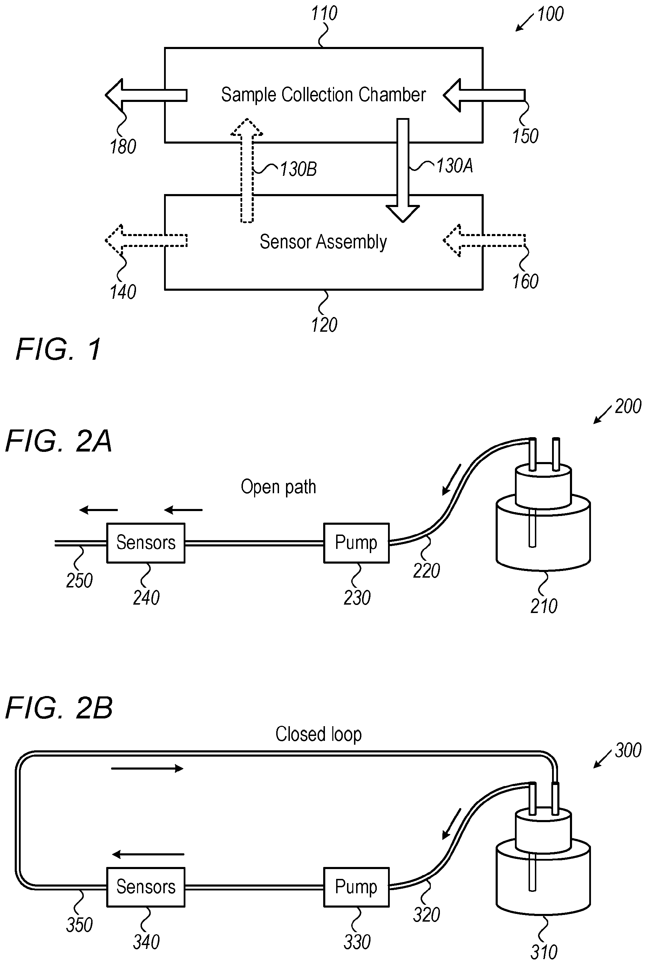

As disclosed herein, a device of the invention is a hand-held device which generally comprises a sample collection region and a sensing region and the device is configured to permit flowing of a sample from the sample collection region onto the sensing region or directly from an inlet of the device onto the sensing region. In most general terms, and as is generally depicted in **Fig. 1****,** a device 100 of the invention comprises at least two detachable parts 110 and 120, wherein a first part comprises a sample collection chamber 110 and a second part comprises at least one sensor assembly 120. The sample collection chamber 110 is provided with an inlet 150 and an outlet 180. The sensor assembly section also comprises an inlet 160 and an outlet 140. The two sections are associated with each other via a channel assembly 130A that is configured to direct a sample contained in said sample collection chamber 110 to the at least one sensor assembly 120. The sample thereafter may exit the sensing assembly section through outlet 140 or may be flown back into the sample collection chamber via outlet 130B and circulated back once and again. Not shown in **Fig. 1** are means for circulating said sample over the at least one sensor assembly, said means being a pump. An analyzer configured for real-time analysis of the VC content in the sample is also provided in a device 100.

Sections 110 and 120 of device 100 are detachable and one or both may be disposable.

A device of the invention may comprise a plurality of valves which separate between the sections and which permit directional flow of the sample from the sample collection chamber 110 to the sensor assembly 120. Sample flown over the sensor assembly 120 may thereafter exit the sensor via outlet 140 or recirculated via outlet 130B and inlet 130A. The two circulation modes are illustrated in **Figs. 2A** and **2B** which depict an experimental set-up demonstrating operation of a device in detecting and quantifying an analyte in a gaseous medium.

As exemplified in **Fig. 2A****,** a device 200 operated in an open circulation mode is depicted. In such a configuration, a sample is flown from the sample collection chamber 210 through a channel assembly or a conduit 220 and a pump 230 into a sensor assembly section 240. After flowing over the assembly 240, the sample exits the device via outlet 150. Device 300, shown in **Fig. 2B****,** is configured to operate as a closed loop circulation mode. In this configuration, the same sample may be circulated and recirculated over the sensor assembly. A sample may be flown from the sample collection chamber 310 through a channel assembly or a conduit 320 and a pump 330 into a sensor assembly section 340. After flowing over the assembly 340, the sample recirculates via outlet 350 into the chamber 310 from which it is recirculated over the sensor assembly.

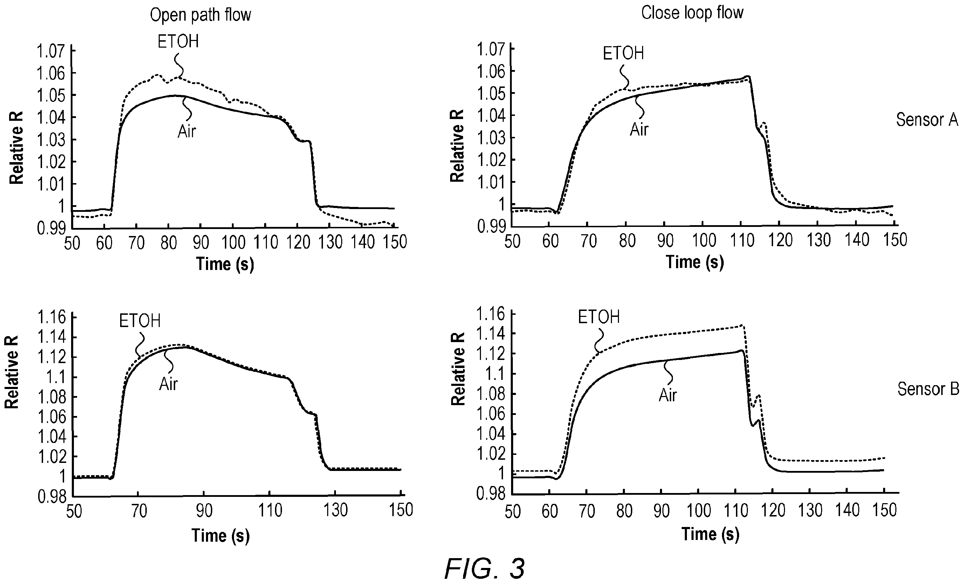

In an exemplary run, a 1 ml sample of 7% ethanol in distilled water was used to mimic a gaseous sample. The headspace of ethanol vapors generated in the sample container were pumped and circuited over the sensors chamber. In one setup the headspace was pumped in an open circulation mode and in another setup the headspace was pumped in a closed loop circulation mode. Ethanol vapors were pumped through the sensors for approximately 1-2 min. Before and following circulation with the ethanol vapors, clean air was circulated over the sensors. As shown in **Fig. 3****,** in the open circulation mode, the initial reaction to the vapors was observed, but immediately following the initial signal, the signal decreased due to dilution of the sample with air from the surroundings. In the closed loop circulation mode, however, a complete signal was observed - initial phase of rapid signal increase followed by a slow increase towards signal stabilization.

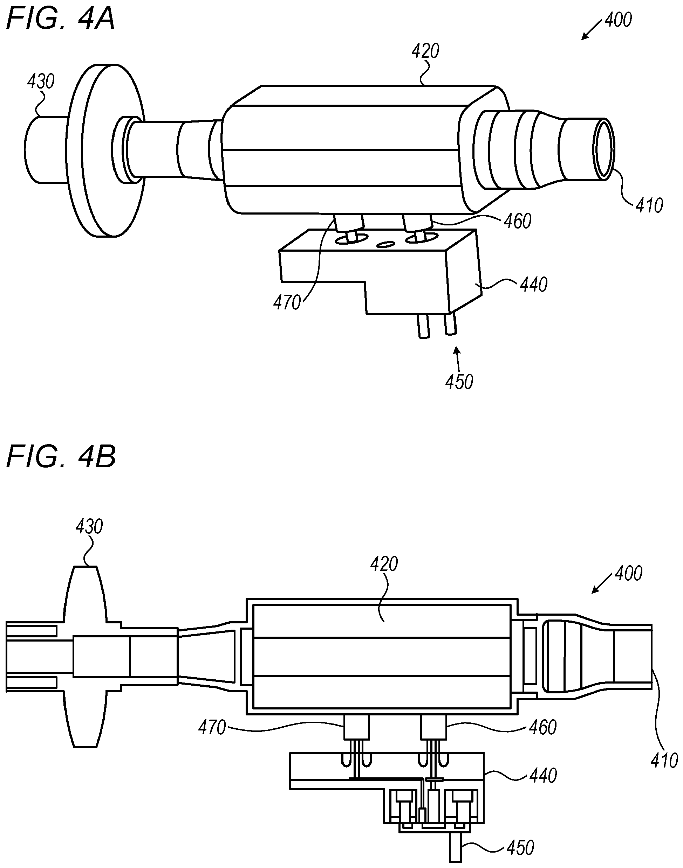

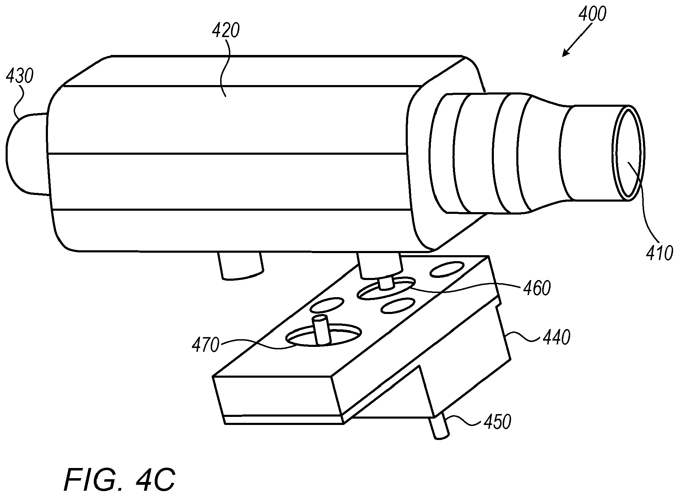

**Fig. 4** provides another embodiment of a device of the invention. In **Fig. 4A****,** a device 400 is shown having a sample collecting chamber 420 adapted with an inlet 410 and an outlet 430. The sensor assembly section 440 may be adapted with a pump via inlet 450 and associated with the sample collection chamber via inlet 460 and outlet 470. The configuration depicted in **Fig. 4A** permits continuous sample recycling via inlet and outlet 460 and 470, respectively, as disclosed herein. The configuration shown in **Fig. 4C****,** however, having outlet 470 disconnected from the sample collecting chamber, permit the open circulation mode, whereby a sample flown over the sensor assembly exits the device without being sent back into the sample collecting chamber.

**Fig. 4B** provides a cross section view of a device 400 according to the invention. Each of the elements is as depicted in **Fig. 4A****.**

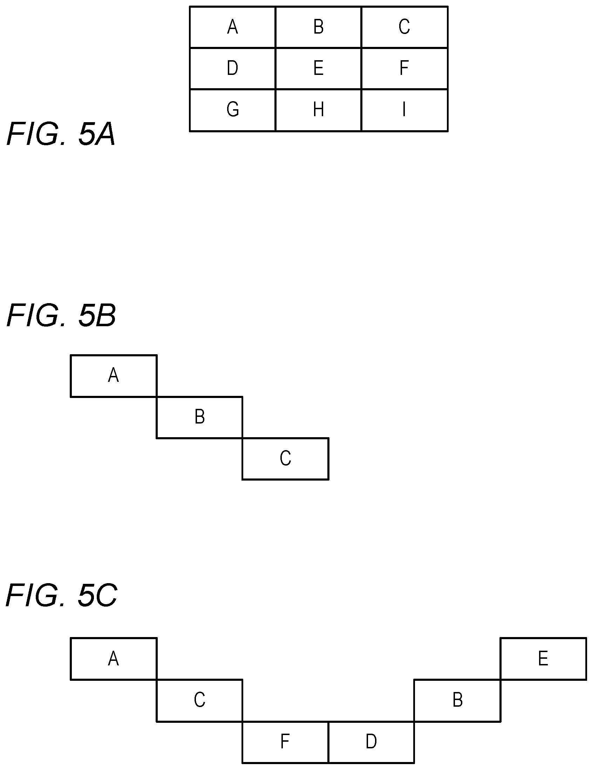

An exemplary sensor assembly disclosed herein is depicted in **Fig. 5****.** As a person versed in the other, the illustrated assembly may be structured in different ways to achieve sensitivity towards the various VCs. As shown in **Fig. 5****,** a sensor assembly comprises of a plurality of sensing regions, wherein each of the regions may be associated with a different sensor moiety type or population, as disclosed herein. In **Fig. 5A** a square assembly is provided wherein 9 sensing regions are structured as shown. Each of the sensing regions A, B, C, D... I may comprise the same or different sensors, each may comprise a homogenous population of different sensors, a heterogeneous population of sensors or each may comprise the same sensors. In **Figs. 5B** and **5C****,** different assemblies or arrays are provided, wherein each of the sensing regions may be selected as above.

The sensor assembly comprises a plurality of sensing regions A, B, C... etc, wherein each of the regions is provided with a plurality of nanoparticles that are associated to the surface. Each of the regions A, B, C... etc is associated with same or different population of nanoparticles, such that a signal may be independently derived from each of the sensing areas, and be indicative of an interaction (or lack thereof) between VCs present in the sample and the nanoparticles on the sensing regions. Different nanoparticles may vary in particle size, particle morphology (e.g., core/shell particles, non-core/shell particles, spherical, cubic, tetrahedral, triangular, dumbbell, elongated, multiparticles or fused particles, etc), particle composition (e.g., doping, metallic particles, non-metallic particles, conductive particles, novel metal particles, hybrid materials, etc), surface decoration (e.g., presence of material islands, association with ligand groups, etc) and others.

Nanoparticles surface decoration may be used, in some configurations, to differentiate between nanoparticle populations. Typically, nanoparticles are surface associated or decorated with a plurality of VC interacting ligand molecules. The interaction being chemical or physical allows for a measurable change which can be detected and analyzed to yield an indication of presence and/or amount of a VC in a gaseous sample. In some instances and despite presence of the ligand molecules, the measurable interaction is between the VC and the nanoparticles surface. The ligand molecules may be selected, for example, and without limitation, from dodecanethiol, hexanethiol, decanethiol, tert-dodecanethiol, butanethiol, 2-ethylhexanethiol, dibutyl disulfide, 2-nitro-4-trifluoromethylbenzenethiol, benzylmercaptane, 4-chlorobenzenemethanethiol, 3-ethpxythiolphenol, 4-tert-methylbenzenethiol, 1-heptanethiol, 1,8-naphthyridine, 10-undecyn-l-ol, 3- methyl-1 -butanol, 1-phenyl-lH-imidazole, 2-(2-pyridinyl)- lH-indole, 8-methyl-lH- purine, 1-methoxyphthalazine, 1-nitro-2-propanol, 2-butyl- 1-octanol, 3,7-dimethyl-loctanol, 2-methyl- 1-propanol, 1-undecene, 2-(2-methylpropyl)-3,5-di(l-methylethyl)pyridine, 2,3-dimethylcyclohexylamine, 2,4-dithiapentane, 2-benzyl-1-methylpiperidine, 2-butanone, 3-propylidene-2-heptanone, 6-phenylhexanoic acid, 8- aminocaprylic acid, 2,2'-thiobis-acetic acid, acetone, O-isopropyloxime benzaldehyde, 4-nitro-benzamide, 2-carboxy-benzeneacetic acid, 2-methyl-butanal, 3-methyl-butanal, 3-methyl-butanoic acid, dodecamethyl-cyclohexasiloxane, cyclohexene, dimethyl trisulfide, dimethyl disulfide, ethylphenylhydantoin, gabapentin lactam, ethyl 5-oxohexanoate, 5-nitro-isoquinoline, lanostan-12-one, luminol (5-amino-2,3- dihydrophthalazine-l,4-dione), 4-butyl-phenol, phthalic anhydride, pregabalin, 1-(ethynylsulfinyl)-propane, S-(2-benzothiazolyl)cysteine, 2-butyl5-ethyl-thiophene, cyclopropyl carbinol, 2-pyridinecarbonitrile, 2-bromo-l-(4-methylphenyl)-ethenone, 2,3,4,7-tetrahydro-lH-indene, 1-bromo-l-phenylpropane, 2,6- dimethyldecane, N,N-dimethyl-l-dodecanamine, 3-methyl-6-(l-methylethylidene)- cyclohexene, 8-methyl- 1-decene, 6-methyl-dodecane, N,N-dimethyl-l-tetradecanamine, hexanedioic acid bis(2-ethylhexyl) ester, (+)-4-carene, 2-carene, 2-methyl-1-propene, 3-methylpentan-2-yl trifluoroacetate, 2-methyl-5-(l-methylethenyl)-cyclohexanol, (Z)-4-Decen-1-ol trifluoroacetate, 4-methyl-l-(l-methylethyl)-bicyclo[3.1.0]hexan-3-ol, pyruvic acid butyl ester, 2,9-dimethyl-decane, propylamine, ethylenediamine, l-methyl-2-(3-methylpentyl)-cyclopropane, (nitromethyl) benzene, 5-ethyl-1-nonene, isopropylsulfonyl chloride, 2,3,6,7-tetramethyl-octane, 1-methyl-4-(lmethylethenyl)-benzene, 3,4-dimethyl-1-pentene, N-bcnzyl-N-methyl-2-methyl-alanine methyl ester, 2,2,4-trimethyl-pentane, trans-geranylgeraniol, 2-ethyl-4-methyl1-pentanol, 6-methylheptyl vinyl ether, tetrahydro-6-methyl-2H-pyran-2-one, 2,3,7- trimethyl-decane, 2-decen-l-ol, (lR,4aS,8aR)-l-isopropyl-4,7-dimethyl-1,2,4a,5,6,8ahexahydronaphthalene, 3-ethyl-2-methyl-hexane, 2-methyl-1-pentene, 4,5-dimethylundecane, 4-methylene-1-methyl-2-(2-methyl-1-propen-1-yl)-l-vinyl-cycloheptane, 7-methyl-(E)-4-decene, 1-iodo-dotriacontane, 5-dodecyldihydro-2(3H)-furanone, butyl dodecyl ester sulfurous acid, 3 4-dimethylbenzyl alcohol, 1,4-dimethyl-cyclooctane, 2,3- dimethyl-hexane, dodecanoic acid, estragole, 4-ethyl-l-octyn-3-ol, 5-methyl-2-(lmethylethyl)-l-hexanol, 3,3-dimethyl-heptane, 7-methyl-(Z)-2-decene, 2-methyl-decane, l,2,3,4,4a,5,6,7,8,9,10,10a-dodecahydro-l,4a-dimethyl-7-(l-methylethyl)-l-phenanthrenecarboxylic acid methyl ester, dodecanal, 1-octadecanesulphonyl chloride, 4- tert-butylcyclohexyl acetate, 4-hexen-2-one, 2,5,6-trimethyl-decane, 4 4-dimethyl-1- hexene, heptadecane, isobutylene epoxide, 2,2,7,7-tetramethyloctane, 2-ethyl- 1-hexanol trifluoroacetate, propylcyclopropane, anethole, octane, methyl-cyclobutane, 1,12-dodecanediol, 2-methoxy-l-propene, nitrous acid, 4-(1,1-dimethylethyl)cyclohexanol acetate, l,5-dimethyl-8-(l-methylethylidene)-(E,E)-5-cyclodecadiene, 4-methyl-2- propyl- 1-pentanol, octahydro-4-methyl- 8-methylene-7-( 1-methylethyl)-[1Sα,3β,4α,7α,7a]-4-methano-H-indene, 3-ethyl-2, 7-dimethyl-octane, hexyl pentyl ether, 1,2-diphenyl-(R*,R*)-1,2-ethanediol, 7-ethyl-l,2,3,4,4a,5,6,7,8,9,10,10adodecahydro-1,4a,7-trimethyl-methyl ester [1S-(1 ,4a,7,1 β)]-1- phenanthrenecarboxylic acid.

The number of sensing regions in a sensing assembly is more than 1. The number of regions is, for example, 2, 3, 4, 5, 6, 7, 8, 9, 10, 11, 12,13, 14, 15, 16, 17, 18, 19 or 20 or more.

## Claims

1. A hand-held device for determining presence of a volatile compound (VC) in or content of at least one gaseous sample, the device comprising at least two detachable parts (110, 120), wherein a first part comprises a sample collecting chamber (110); and a second part comprises at least one sensor assembly (120); wherein
the sample collecting chamber (110) and the at least one sensor assembly (120) are in gaseous communication;
the device further comprising a closed loop channel assembly (130A) and a pump (230) configured to direct said sample from the sample collecting chamber (110) to the at least one sensor assembly (120) and to circulate said sample from the sample collecting chamber (110) over the at least one sensor assembly (120) over a period of time; wherein the circulation is continuous back to and from the collection chamber (110) and over the sensor assembly (120) to maintain a stable and continuous measurement; and
an analyzer configured for real-time analysis of the VC presence in or content of the sample; and a data processing unit for data communication with the sensor assembly; a data user interface unit being in data communication with the data processing unit; wherein the data processing unit comprising data relating to a control data set and is adapted to receiving from the at least one sensor assembly information relating to presence of VCs or pattern thereof and provide an indication of presence or absence of one or more VCs, and disease state
wherein the at least one sensor assembly (120) comprises a plurality of sensing regions and wherein optionally one or more of said at least two detachable parts are disposable, wherein
each of the plurality of sensing regions is provided with a plurality of nanoparticles associated to a surface of each of the plurality of sensing regions and wherein the nanoparticles are surface-associated with a plurality of VC interacting ligand molecules.

2. The device according to claim 1, wherein the sample is a breath sample.

3. The device according to claim 2, wherein the breath sample is obtained from a subject and is received through an inlet provided in the sample collecting chamber (110).

4. The device according to claim 1, comprising two or more sample collecting chambers, wherein one or more of the sample collecting chambers is an environment testing chamber adapted with one or more sensors providing an initial reading of environmental parameters.

5. The device according to claim 4, wherein the one or more sensors are configured for providing a reading relating to any one of gas composition, carbon dioxide presence and concentration, humidity and sample temperature.

6. The device according to claim 1, wherein one or more of said at least two detachable parts is disposable.

7. The device according to any one of the preceding claims, wherein the VCs are associated with the metabolism, presence and/or growth of at least one pathogen or involved in the pathogenesis of a disease or disorder.

8. The device according to any one of the preceding claims, for determining a VC profile selective and indicative of onset, evolution or progression of a disease state.

9. The device according to claim 8, wherein the VC profile differentiating one disease over another, provides an indication of a disease state or progression thereof, identifies onset of a disease at a stage before symptoms develop and/or determine success of a therapeutic treatment.

10. The device according to claim 1, wherein the sample collecting chamber (110) is configured to receive a gaseous sample while disconnected from the at least one sensor assembly (120).

11. The device according to claim 1, wherein the closed loop channel assembly (130A) has at least one outlet operable to exhaust the sample upon demand.

## Patentansprüche

1. Handvorrichtung zum Feststellen des Vorhandenseins einer flüchtigen Verbindung (VC) in oder als Inhalt von mindestens einer gasförmigen Probe, wobei die Vorrichtung mindestens zwei lösbare Teile (110, 120) umfasst, wobei ein erstes Teil eine Probensammelkammer (110) umfasst und ein zweites Teil mindestens eine Sensoranordnung (120) umfasst, wobei
die Probensammelkammer (110) und die mindestens eine Sensoranordnung (120) in Gasaustausch sind;
die Vorrichtung ferner eine Kanalanordnung (130A) mit geschlossenem Kreislauf und eine Pumpe (230) umfasst, die dazu konfiguriert ist, die Probe von der Probensammelkammer (110) zu der mindestens einen Sensoranordnung (120) zu leiten und über einen Zeitraum die Probe von der Sensorsammelkammer (110) über die mindestens eine Sensoranordnung (120) zu zirkulieren; wobei die Zirkulation kontinuierlich zurück zu und von der Sammelkammer (110) und über die Sensoranordnung (120) erfolgt, um eine stabile und kontinuierliche Messung aufrechtzuerhalten; und
einen Analysator, konfiguriert für eine Echtzeitanalyse auf das Vorhandensein einer VC in oder als Gehalt der Probe; und eine Datenverarbeitungseinheit für Datenaustausch mit der Sensoranordnung, eine Datenbenutzerschnittstelleneinheit, die in Datenaustausch mit der Datenverarbeitungseinheit ist, wobei die Datenverarbeitungseinheit Daten in Bezug auf einen Steuerungsdatensatz umfasst und dazu ausgelegt ist, von der mindestens einen Sensoranordnung Informationen im Zusammenhang mit dem Vorhandensein von VCs oder einem Muster davon zu empfangen und einen Hinweis auf das Vorhandensein oder Fehlen einer oder mehrererVCs und eines Krankheitszustands bereitzustellen,
wobei die mindestens eine Sensoranordnung (120) eine Vielzahl von Abtastbereichen umfasst und wobei optional eines oder mehrere der mindestens zwei lösbaren Teile wegwerfbar sind, wobei
jede von der Vielzahl von Abtastbereichen mit einer Vielzahl von Nanopartikeln versehen ist, die einer Oberfläche jeder von der Vielzahl von Abtastbereichen zugeordnet sind, und wobei die Nanopartikel mit einer Vielzahl von mit VC interagierenden Ligandenmolekülen oberflächenassoziiert sind.

2. Vorrichtung nach Anspruch 1, wobei die Probe eine Atemprobe ist.

3. Vorrichtung nach Anspruch 2, wobei die Atemprobe von einem Subjekt erhalten wird und durch einen in der Probensammelkammer (110) bereitgestellten Einlass aufgenommen wird.

4. Vorrichtung nach Anspruch 1, umfassend zwei oder mehrere Probensammelkammern, wobei eine oder mehrere der Probensammelkammern eine Umgebungsprüfkammer ist, angepasst mit einem oder mehreren Sensoren, die einen Anfangsmesswert von Umgebungsparametern bereitstellen.

5. Vorrichtung nach Anspruch 4, wobei der eine oder mehrere Sensoren dazu konfiguriert sind, einen Messwert in Bezug auf eine Gaszusammensetzung, das Vorhandensein und die Konzentration von Kohlendioxid, die Feuchtigkeit und/oder die Probentemperatur bereitzustellen.

6. Vorrichtung nach Anspruch 1, wobei eines oder mehrere der mindestens zwei lösbaren Teile wegwerfbar sind.

7. Vorrichtung nach einem der vorhergehenden Ansprüche, wobei die VCs mit dem Stoffwechsel, dem Vorhandensein und/oder dem Wachstum mindestens eines Pathogens zusammenhängen oder in die Pathogenese einer Erkrankung oder Störung involviert sind.

8. Vorrichtung nach einem der vorhergehenden Ansprüche zur Bestimmung eines VC-Profils, das selektiv und bezeichnend für das Einsetzen, die Entwicklung oder das Fortschreiten eines Krankheitszustands ist.

9. Vorrichtung nach Anspruch 8, wobei das VC-Profil eine Erkrankung gegenüber einer anderen differenziert, einen Hinweis auf einen Erkrankungszustand oder dessen Voranschreiten bereitstellt, das Einsetzen einer Erkrankung in einer Stufe abgibt, bevor sich Symptome entwickeln, und/oder den Erfolg einer therapeutischen Behandlung definiert.

10. Vorrichtung nach Anspruch 1, wobei die Probensammelkammer (110) dazu konfiguriert ist, eine gasförmige Probe zu empfangen, während sie von der mindestens einen Sensoranordnung (120) getrennt ist.

11. Vorrichtung nach Anspruch 1, wobei die Kanalanordnung (130A) in Form eines geschlossenen Kreislaufs mindestens einen Auslass aufweist, der dazu betreibbar ist, die Probe auf Anforderung abzugeben.

## Revendications

1. Dispositif portatif en vue de la détermination de la présence d'un composé volatil (VC) dans ou comme contenu d'au moins un échantillon gazeux, le dispositif comprenant au moins deux parties détachables (110, 120), dans lequel une première partie comprend une chambre collectrice d'échantillons (110) ; et une deuxième partie comprend au moins un ensemble de capteurs (120) ; dans lequel
la chambre collectrice d'échantillons (110) et au moins l'un des ensembles de capteurs (120) sont en communication gazeuse ;
le dispositif comprend par ailleurs un ensemble de canaux en boucle fermée (130A) et une pompe (230) configurés afin de diriger ledit échantillon de la chambre collectrice d'échantillons (110) vers au moins l'un des ensembles de capteurs (120) et afin de faire circuler ledit échantillon de la chambre collectrice d'échantillons (110) via au moins l'un des ensembles de capteurs (120) sur une période de temps ; dans lequel la circulation est continue au retour de et à partir de la chambre collectrice (110) et via l'ensemble de capteurs (120) afin de maintenir une mesure stable et continue ; et
un analyseur configuré en vue de l'analyse en temps réel de la présence de VC dans ou comme contenu de l'échantillon ; et une unité de traitement des données en vue de la communication de données avec l'ensemble de capteurs; une unité d'interface utilisateur de données étant en communication de données avec l'unité de traitement des données ; dans laquelle l'unité de traitement des données comprend des données relatives à un jeu de données de contrôle et est adaptée à recevoir de la part d'au moins l'un des ensemble de capteurs des informations relatives à la présence de VCs ou à une configuration de ceux-ci et à fournir une indication de la présence ou de l'absence d'un ou de plusieurs VCs, et de l'état de la pathologie
dans lequel au moins l'un des ensembles de capteurs (120) comprend une pluralité de régions de détection et dans lequel, en option, une ou plusieurs desdites au moins deux parties détachables sont jetables, dans lequel
chacune de la pluralité de régions de détection est dotée d'une pluralité de nanoparticules associées à une surface de chacune de la pluralité de régions de détection et dans laquelle les nanoparticules sont associées en surface à une pluralité de molécules de ligand interagissant avec le VC.

2. Dispositif selon la revendication 1, dans lequel l'échantillon est un échantillon d'haleine.

3. Dispositif selon la revendication 2, dans lequel l'échantillon d'haleine est obtenu d'un sujet et est reçu à travers une entrée présente dans la chambre collectrice d'échantillons (110).

4. Dispositif selon la revendication 1, comprenant deux chambres collectrices d'échantillons ou plus, dans lequel une ou plusieurs des chambres collectrices d'échantillons sont une chambre de test environnemental adaptée avec un ou plusieurs capteurs présentant une lecture initiale des paramètres environnementaux.

5. Dispositif selon la revendication 4, dans lequel un ou plusieurs capteurs sont configurés en vue de la livraison d'une lecture relative à une propriété quelconque parmi la composition du gaz, la présence et la concentration de dioxyde de carbone, l'humidité et la température d'échantillon.

6. Dispositif selon la revendication 1, dans lequel une ou plusieurs desdites au moins deux parties détachables sont jetables.

7. Dispositif selon une quelconque des revendications précédentes, dans lequel les VC sont associés au métabolisme, à la présence et/ou à la croissance d'au moins un agent pathogène ou impliqués dans la pathogenèse d'une pathologie ou d'un trouble.

8. Dispositif selon une quelconque des revendications précédentes, en vue de la détermination d'un profil de VC sélectif et indicateur de l'apparition, de l'évolution ou de la progression d'un état pathologique.

9. Dispositif selon la revendication 8, dans lequel le profil de VC distinguant une pathologie d'une autre fournit une indication d'un état pathologique ou de la progression de celui-ci, identifie l'apparition d'une pathologie à un stade antérieur à l'apparition des symptômes et/ou détermine le succès d'un traitement thérapeutique.

10. Dispositif selon la revendication 1, dans lequel la chambre collectrice d'échantillons (110) est configurée afin de recevoir un échantillon gazeux pendant sa déconnexion d'au moins l'un des ensembles de capteurs (120).

11. Dispositif selon la revendication 1, dans lequel l'ensemble de canaux en boucle fermée (130A) possède au moins une sortie actionnable afin d'évacuer l'échantillon sur demande.
